# EUROPÄISCHE PATENTANMELDUNG

(11) **EP 0 984 255 A1**
(43) Veröffentlichungstag der Anmeldung: **08.03.2000**
(21) Anmeldenummer: 99117165.3
(22) Anmeldetag: 01.09.1999
(51) Int. Cl.: G01K 13/00, A61B 5/00

(54) **Vorrichtung und Verfahren zur berührungslosen Erfassung einer Oberflächentemperatur**

(30) Priorität: 02.09.1998 DE 29815618 U
(71) Anmelder: Steinel GmbH & Co. KG, 33442 Herzebrock-Clarholz (DE)
(72) Erfinder: Meggle, Martin, 33442 Herzebrock-Clarholz (DE)
(74) Vertreter: Behrmann, Niels, Dipl.-Ing.

(57) **Zusammenfassung**

Die Erfindung betrifft eine Vorrichtung zur berührungslosen Temperaturerfassung einer Oberflächentemperatur, insbesondere der menschlichen Haut, mit einer zum berührungslosen Erfassen einer Temperatur als Reaktion auf ein Startsignal sowie zum Ausgeben eines entsprechenden Temperatursignals ausgebildeten, berührungslosen Messeinheit, wobei die handbetätigt, portabel und netzunabhängig betreibbare Messeinheit zum bevorzugt drahtlosen Übertragen mindestens eines Temperatursignals eine weiter bevorzugt auf Infrarotbasis wirkende Sendereinheit aufweist, die Sendereinheit zum Zusammenwirken mit einer stationären, mit einer Datenverarbeitungsvorrichtung verbundenen Empfangseinheit ausgebildet ist und die Datenverarbeitungseinheit zum Auswerten einer Mehrzahl von digitalisierten Temperatursignalen als Reaktion auf ein ein Ende eines Messbetriebs bestimmendes Steuersignal ausgebildet ist.

## Beschreibung

Die vorliegende Erfindung betrifft eine Vorrichtung zur berührungslosen Temperaturerfassung einer Oberflächentemperatur nach dem Oberbegriff des Patentanspruches 1.

Vorrichtungen zur berührungslosen Temperaturerfassung sind aus dem Stand der Technik bekannt, wobei sich handelsübliche, entweder auf dem pyroelektrischen Prinzip basierende Temperatursensoren (sog. Pyro-Sensoren) oder auf dem thermoelektrischen Prinzip basierende Temperatursensoren (sog. Thermopilesensoren), bevorzugt zur berührungslosen Temperaturmessung von Objekten eignen. Beide Sensortypen werden etwa von der Firma Heimann Optoelectronic, Wiesbaden, angeboten.

Genauer gesagt basiert der pyroelektrische Effekt auf der durch Temperaturänderung bedingten Änderung der elektrischen Ladung in einem Kristall. Somit kann mit derartigen Sensoren nur mittels eines, in der Regel mechanischen, Choppers ein Temperatursignal relativ zur Umgebungstemperaur des Sensors ermittelt werden. Handelsübliche Ohrthermometer verwenden beispielsweise derartige Prinzipien. Der thermoelektrische Effekt beruht hingegen, als Pendant zum Peltier-Effekt, auf dem Prinzip der temperaturabhängigen Spannungserzeugung durch üblicherweise eine Mehrzahl von in Reihe geschalteten Thermoelementen, wobei ein handelsübliches Thermopile-Sensorelement aus einem halbleitenden, bevorzugt polykristallinen Siliziumsubstrat als Halbleitermaterial besteht, auf welchem eine dünne Membran einer niedrigen Temperaturleitfähigkeit aufgebracht ist. Auf dieser Membran ist dann eine Mehrzahl von Thermoelementen von zur Signalerzeugung gebildet, wobei die Gesamtanordnung durch spezielle Maßnahmen im Infrarot-Bereich (IR) sensitiv ist. Über dem sog. Thermopile-Array befindet sich eine sog. Schwarzschicht zu Absorption von Wärmestrahlung. Die Erwärmung dieser Schicht wird vom Thermopile-Array gemessen. Im Ergebnis misst ein solcher, gängiger Thermopile-Sensor eine einfallende Strahlungstemperatur als Relativwert zur Umgebungstemperatur des Sensors.

Thermopile Sensoren liefern zwar ein kleineres Ausgangssignal als pyroelektrische Sensoren, dafür benötigen sie jedoch keinen mechanischen Chopper.

Während derartige berührungslose Sensorvorrichtungen mit zugehöriger Signalauswertelektronik etwa in der industriellen Messtechnik bereits einige Anwendungen besitzen, so hat sich jedoch bislang, insbesondere aufgrund der Problematik der notwendigen Berücksichtigung des Emissionsgrades einer zu messenden Oberfläche, diese Meßtechnik im Consumer-Bereich als aufwendig und schwierig in der Realisierung erwiesen.

Darüber hinaus ist für genaue Messungen einer Absoluttemperatur, wie es etwa im medizinischen Bereich bei der Fiebermessung der Fall ist, eine berührungslose Temperaturerfassung ebenfalls nur mit sehr hohem Aufwand realisierbar, da insbesondere im Feld (d.h. außerhalb einer Laborumgebung) eine Reproduzierbarkeit von absoluten Temperaturwerten im Zehntel Grad-Bereich schwierig ist.

Aufgabe der vorliegenden Erfindung ist es daher, eine Vorrichtung zur berührungslosen Temperaturerfassung nach dem Oberbegriff des Anspruches 1 zu schaffen, die hinsichtlich Bedienung, Auswertbarkeit der gemessenen Temperaturwerte und Anbindung an Systeme zur automatischen (Mess-) Datenverarbeitung weiterentwickelt ist, sich insbesondere auch für medizinische Anwendungen bzw. einen Einsatz in einem medizinischen, klinischen Umfeld eignet, und mit geringem systemtechnischen Aufwand realisierbar ist.

Die Aufgabe wird durch die Vorrichtung mit den Merkmalen des Patentanspruches 1 gelöst.

Dabei ist als berühungslose Messeinheit jegliche Vorrichtung zu verstehen, die mit mindestens einem berührungslos arbeitenden Sensor, beispielsweise einem Thermopile-Sensor, wie von der Firma Heimann angeboten, sowie einer nachgeschalteten Auswerteelektronik zum Erzeugen des Temperatursignals versehen ist.

Ferner sind als Sendereinheit bzw. Empfangseinheit im Rahmen der Erfindung solche Vorrichtungen zu verstehen, die zwar bevorzugt auf drahtloser Basis Temperatursignale in analoger oder digitaler Weise übertragen, gleichwohl sollen jedoch auch solche Signal- bzw. Datenübertragungseinheiten als Sender- bzw. Empfangseinheit verstanden werden, bei denen etwa im Wege einer drahtgebundenen Übertragung, z.B. einem Kontakt zwischen Handgerät und Basisstation, oder aber einem Schnittstellenkabel, die Übertragung der Temperatursignale erfolgen kann.

In erfindungsgemäß vorteilhafter Weise ermöglicht es die Vorrichtung, auf komfortable, bedienungssichere Weise eine Mehrzahl von Temperaturwerten verschiedener, zugeordneter Oberflächen, insbesondere auf einem menschlichen Oberkörper, zu erfassen und einer zentralen Datenauswertung zuzuführen. Dabei liegt ein wesentlicher Anwendungsfall im Bereich der medizinischen Messdatenerfassung durch Untersuchung der menschlichen Haut, etwa zur Feststellung von Gelenkentzündungen od.dgl. Zuständen, indem eine Mehrzahl vorbestimmter Oberflächenpunkte auf der menschlichen Haut durch eine Bedienperson der handbetätigten, portablen Einheit sukzessive abgetastet wird und dann die erfaßten Temperatursignale einer zentralen Weiterverarbeitung, insbesondere mittels eines handelsüblichen PC, zur Verfügung stehen.

Vorteilhafte Weiterbildungen der Erfindung sind im weiteren bzw. in den Unteransprüchen beschrieben.

So ist es besonders bevorzugt, dass die handbetätigte Messeinheit entsprechend einer Mehrzahl von auf zunehmenden Oberflächenmesspunkten eine Speichervorrichtung aufweist, die, über das Speichern der jeweiligen, zugehörigen Temperatursignale hinaus, auch dem Bediener einen jeweils folgenden Messpunkt mittels einer weiterbildungsgemäß vorgesehenen Anzeige- bzw. Ausgabeeinheit signalisieren kann.

Weiterbildungsgemäß weist hierzu die Messeinheit eine bevorzugt numerische oder alphanumerische Anzeigeeinheit auf, die zum Anzeigen eines aktuellen, erfassten Temperaturwertes und/oder einer Messpunktbezeichnung und/oder einer Nummer eines Messvorganges für eine Bedienperson ausgebildet ist, wobei, weiter bevorzugt, die Anzeigeeinheit zum umschaltbaren Anzeigen der erfassten Temperatur in °C und °Fahrenheit als Reaktion auf eine manuelle Betätigung eines Schaltelementes ausgebildet ist.

Die weiterbildungsgemäß vorgesehene Pufferspeichereinheit dient darüber hinaus dazu, manuell schwer zugängliche Messpunkte mit ihrem zugehörigen Temperatursignal auf Knopfdruck durch die Bedienperson festzuhalten.

Bevorzugt weist zu diesem Zweck die berührungslos arbeitende Messeinheit einen Pufferspeicher zum Halten mindestens eines Temperatursignals als Reaktion auf eine manuelle Betätigung eines etwa an einem Gehäuse der Messeinheit vorgesehenen Betätigungsknopfes auf, wobei, weiter bevorzugt als Reaktion auf eine manuelle Betätigung eines Bedienknopfes ein im Pufferspeicher gehaltenes Temperatursignal in eine für eine Mehrzahl von Temperatursignalen ausgebildete Speichereinheit überführt wird.

Gemäß einer weiteren bevorzugten Ausführungsform der Erfindung ist eine bevorzugt in der berührungslos arbeitenden Messeinheit vorgesehene Einheit zum Einstellen des Emissionsgrades der zu messenden Oberfläche vorgesehen, weiter bevorzugt mit einer Einrichtung zum Speichern einer Mehrzahl manuell auswertbarer Werte für vorbestimmte oder vorbestimmbare Emissionsgrade. Auch können bevorzugt die Messeinheit und/oder die Datenverarbeitungsvorrichtung ein manuell betätigbares Schaltelement zum Löschen eines erfassten Temperaturwerts aufweisen.

Weiterbildungsgemäß und besonders vorteilhaft ist es zudem, die Messeinheit drahtlos, etwa mittels einer Infrarot-Strecke, an eine die Empfangseinheit aufnehmende Basisstation anzukoppeln, wobei diese Basisstation mittels entsprechender elektrischer Kontakte oder einer Wirbelstromladeeinrichtung (die Energieübertragung erfolgt hier über elektromagnetische Felder) zur portablen Messeinheit zusätzlich dort vorgesehene Akkus zur netzunabhängigen Stromversorgung laden bzw. puffern kann. Insbesondere im Zusammenwirken mit einer solchen Basisstation wird der Bedienungskomfort der erfindungsgemäßen Vorrichtung im klinischen Einsatz beträchtlich erhöht.

Besonders geeignet ist die Empfangseinheit gemäß einer vorteilhaften Ausführungsform der Erfindung mittels einer Standardschnittstelle, insbesondere mit einer seriellen Schnittstelle versehen, die mit einer als konventionellem PC realisierten Datenverarbeitungsvorrichtung verbindbar ist.

Unabhängiger Schutz im Rahmen der vorliegenden Erfindung wird beansprucht für ein Verfahren zur pyroelektrischen Temperaturerfassung einer Oberflächentemperatur, welches die Merkmale der Verfahrensansprüche 8 bis 10 aufweist.

Analog zur erfindungsgemäßen Vorrichtung wird hierdurch, neben den vorbeschriebenen Vorteilen, vor allem auch erfindungsgemäß der Effekt ausgenutzt, dass, im Einsatz als Diagnostikhilfe etwa für Erfindungsherde im menschlichen Körper (z.B. Rheumaknoten) auf der Basis einer Mehrzahl erfasster, vorbestimmter Messpunkte eine absolute Temperaturerfassung als solche nicht notwendig ist, sondern für die spätere Datenauswertung im Rahmen der Erfindung vielmehr auf relative Abweichungen zwischen den verschiedenen Temperatursignalen zugeordneter Messpunkte abgestellt wird (und hierfür weiterbildungsgemäß Auswertemittel vorgesehen sind). Erfindungsgemäß vorteilhaft zeichnet sich hier nämlich die berührungslose Messtechnik dadurch aus, dass die relative Messgenauigkeit zwischen aufeinanderfolgenden Messwerten bzw. Temperatursignalen einer Messreihe außerordentlich präzise ist (Schwankungen des Emissionsgrades der Haut von Patient zu Patient, langsame Änderungen der Umgebungsbedingungen und ähnliches mitteln sich heraus) und sich dadurch auch vorteilhaft für medizinische Diagnosezwecke eignet.

Besonders günstig ist die vorliegende Erfindung -- sowohl die Vorrichtung, als auch das Verfahren zur berührungslosen Temperaturmessung mit dem Zweck der Temperaturdatenerfassung -- in Bereichen des Sports sowie der Augenheilkunde anwendbar: Es hat sich in praktischen Versuchen herausgestellt, dass durch eine Verwendung der erfindungsgemäßen Vorrichtung zur Temperaturerfassung an menschlichen Gelenken durch berührungslose Temperaturmessung der benachbarten Hautoberfläche Daten generiert werden können, aus deren späterer Auswertung Zeitpunkte einer Gelenk-Überbelastung (etwa durch einen spontanen Anstieg der punktuellen Hauterwärmung) gewonnen werden können. Die vorliegende Erfindung bietet also einem Therapeuten (aber auch entsprechend an ihrem eigenen Leistungsvermögen interessierten Sportlern) die Möglichkeit und Grundlage, etwa Belastungsgrenzen im Training zu ermitteln oder durch kontrollierte Übungen Rehabilitationsmaßnahmen od.dgl. zu beschleunigen.

In der Augenheilkunde eignet sich die vorliegende Erfindung besonders gut als Datenerfassungsapparat bzw. -verfahren, um Eingangsgrößen für eine diagnostische Bestimmung von Augenentzündungen od.dgl. vorzunehmen: Es hat sich herausgestellt, dass hierzu eine Augentemperaturmessung mittels der vorliegenden Erfindung in besonders günstiger Weise als Grundlage für eine Vorhersage von Entzündungen od.dgl. pathologischen Zuständen geeignet ist, insbesondere als Schmerzen (als bislang gültiger Indikator) erst mit dem Auftreten einer akuten Entzündung bemerkt werden, während eine bevorstehende Entzündung sich bereits einige Tage vorher in einer aus Messwerten ablesbaren Erhöhung der Augentemperatur herleiten läßt.

Erfindungsgemäß vorteilhaft wird zudem über die berührungslose Messung der Nachteil herkömmlicher, bekannter Augentemperaturmessungen mittels (kontaktgebender) Sonde vermieden, die üblicherweise von betroffenen Personen als unangenehm oder schmerzhaft empfunden werden.

Gemäß einer bevorzugten Weiterbildung der Erfindung sind optische Markierungsmittel an der handbetätigten Messeinheit vorgesehen, die durch Projektion einer Lichtfläche den für die berührungslose Temperaturerfassung maßgeblichen Oberflächenbereich, etwa auf der Haut oder der Hornhaut, angeben. Das für diese Markierungszwecke verwendete Licht weist den zusätzlichen Vorteil auf, etwa bei einer Augentemperaturmessung das Messergebnis nicht durch zusätzliche Erwärmung zu beeinflussen, und zudem findet eine Gefährdung des empfindlichen Organs durch die Beleuchtung nicht statt.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- Fig. 1:: ein schematisches Blockschaltbild der erfindungsgemäßen Vorrichtung zur pyroelektrischen Temperaturerfassung einer Oberflächentemperatur gemäß einer ersten, bevorzugten Ausführungsform;
- Fig. 2:: ein schematisches Blockschaltbild der Basis- bzw. Adaptereinheit in der Ausführungsform gemäß Fig. 1;
- Fig. 3:: ein weiter detailliertes, schematisches Blockschaltbild des Handmessgerätes der Ausführungsform gemäß Fig. 1;
- Fig. 4:: eine erste, ausschnittsweise Draufsicht auf die Anzeigeeinheit des Handmessgerätes mit darunter angeordneten Bedientasten und
- Fig. 5:: eine Ansicht der Anordnung gemäß Fig. 4 mit einer alternativen Anzeigeinformation für eine Bedienperson.

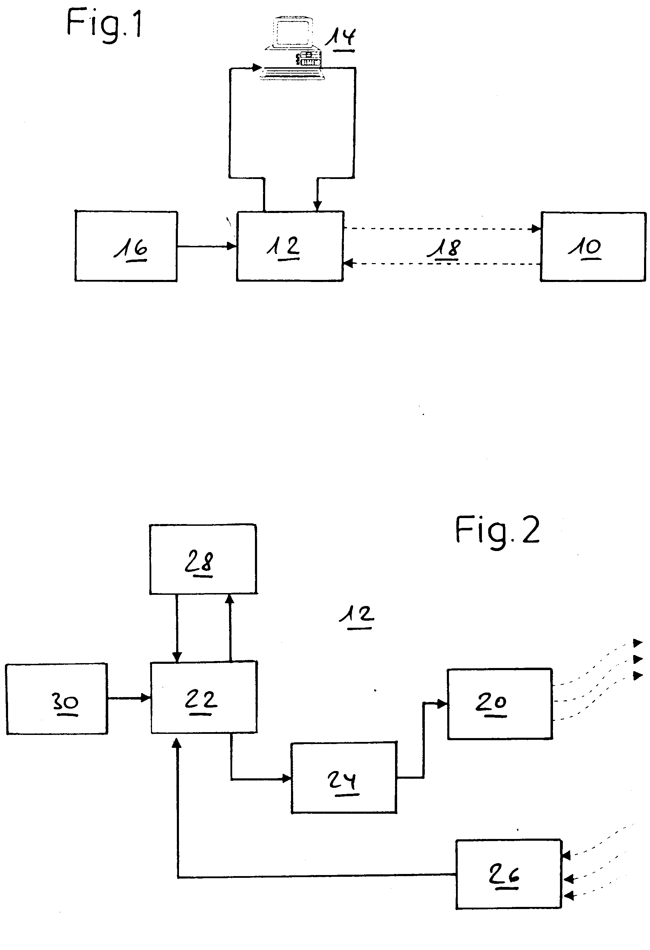

Eine als Basisstation für ein portables, netzunabhängiges Handmessgerät 10 ausgebildete Adaptereinheit 12 bildet im Rahmen der beschriebenen Ausführungsform die Anbindung zwischen einer Datenverarbeitungseinrichtung in Form eines handelsüblichen PC 14 und dem Handmessgerät 10; versorgt durch eine Netzspannungsversorgungseinheit 16 ermöglicht es die Adaptereinheit 12, im aufgelegten Zustand des Handmessgerätes 10 auf die Basisstation 12 Akkumulatoren od.dgl. netzunabhängige Spannungsquellen im Handmessgerät 10 über geeignete Ladekontakte aufzuladen. Darüber hinaus dient die Adaptereinheit 12 zur datentechnischen Anbindung des Handmessgerätes 10 sowohl hinsichtlich vom Messgerät 10 zu übertragender Messdaten, als auch betreffend Positions- bzw. Statusinformationen, die an das Messgerät 10 zur weiteren Ausgabe an eine Bedienperson übertragen werden. In der Darstellung der Fig. 1 ist ein mit dem Bezugszeichen 18 schematisch bezeichneter, drahtloser Datenübertragungspfad auf Infrarot-Basis dargestellt, der insbesondere bei von der Basisstation 12 abgehobenem Handmessgerät 10 eine drahtlose Übertragung von Daten zwischen den Einheiten 10 und 12 (durch ansonsten übliche Infrarot-Technologie) gestattet.

Die Fig. 2 verdeutlicht weiter im Detail den Aufbau der Basis- bzw. Adaptereinheit 12. Zur Realisierung des Datenübertragungspfades 18 enthält die Einheit 12 einerseits eine mit einer geeigneten Infrarot-Sendediode versehene Sendeeinheit 20, die mit von einer Adapterelektronik 22 erzeugten und über eine Verstärkungseinheit 24 verstärkten Sendesignalen zur Weiterleitung an das Handmessgerät 10 versorgt wird. Empfangsseitig weist die Adaptereinheit 12 eine einen geeigneten Infrarot-sensitiven Empfangsstransistor aufweisende Empfangseinheit 26 auf, deren Empfangssignale wiederum der Adapterelektronik 22 zur Aufbereitung und Weiterleitung übermittelt werden.

Zur Kommunikation mit dem PC 14 dient eine mit der Adapterelektronik 22 verbundene Schnittstelleneinheit 28, die im vorliegenden Ausführungsbeispiel als serielle Schnittstelle ausgebildet ist und beispielsweise der RS 232-Norm folgt.

Ein Spannungsversorgungsmodul 30 der Adaptereinheit 12 dient nicht nur der Erzeugung und Aufbereitung der Versorgungsspannung für die in Fig. 2 gezeigten Funktionseinheiten; darüber hinaus bietet das Modul 30 die Ladespannung für die im Handmessgerät 10 vorgesehenen, aufladbaren Batterien an, zur Kontaktierung in einem aufgelegten Zustand des Handmessgerätes.

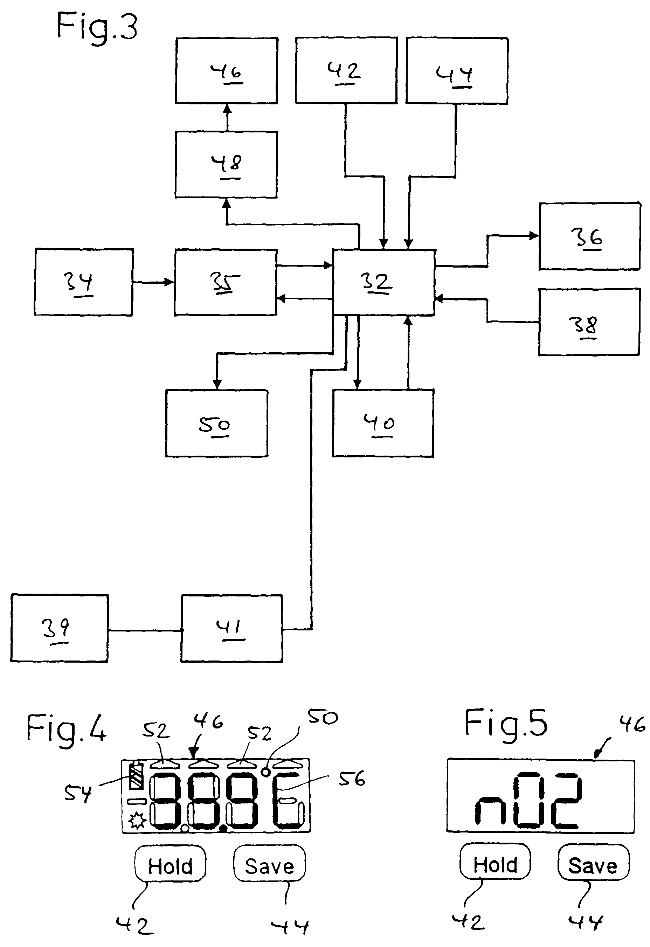

Fig. 3 verdeutlicht den Detailaufbau des Handmessgerätes 10.

Eine zentrale Steuereinheit 32, realisiert mittels eines Mikrocontrollers, übernimmt dabei die Koordination und Betriebssteuerung der weiteren Funktionskomponenten im Handmessgerät.

So ist mit der zentralen Steuereinheit 32 eine Temperatursensoreinheit 34 zur eigentlichen, berührungslosen Temperaturerfassung und zum Erzeugen eines analogen Temperatursignals verschaltet, welches mit einem zwischengeschalteten A/D-Wandler 35 in ein für die Steuereinheit 32 verarbeitbares Digitalsignal gewandelt wird. Zur Kommunikation mit der Basis- bzw. Adaptereinheit 12 ist die zentrale Steuereinheit 32 darüber hinaus sendeseitig mit einer Infrarot-Sendediode 36 bzw. empfangsseitig mit einem Infrarot-Empfangstransistor 38 sowie (nicht gezeigter) Treiberelektronik für diese Elemente verbunden.

Zum Zweck der lokalen Pufferung eines erfassten Messwertes sowie zum (Zwischen-) Speichern einer Mehrzahl von Temperaturmesswerten ist eine Speichereinheit 40 in Form geeigneter, digitaler Speicherbausteine mit der zentralen Steuereinheit 32 verbunden.

Darüber hinaus ist in der Fig. 3 mit dem Bezugszeichen 39 eine ladbare Batterie-/Akkueinheit vorgesehen, die mit einem Spannungsregler 41 zusammenwirkt und die für die Handmesseinheit 10 erforderliche Betriebsspannung zur Verfügung stellt.

Zur Interaktion mit einer Bedienperson sind im Handmessgerät 10 zum einen Anzeigeeinheiten vorgesehen, zum anderen Tasten, die, von der Bedienperson betätigbar, Steuerbefehle geben. Konkret weist das Handmessgerät eine erste Taste 42, die im Schwerpunkt als Haltetaste benutzt wird, sowie eine zweite Taste 44 (die bevorzugt als Speichertaste benutzt wird) auf. Ferner ist als visuelle Schnittstelle zum Benutzer eine mehrstellige LCD-Anzeige 46 mit zusätzlichen, steuerbaren Anzeigesymbolen vorgesehen, welche über eine Treibereinheit 48 angesteuert und mit der zentralen Steuereinheit 32 verbunden ist. Darüber hinaus existiert zum Anzeigen eines aktuellen Messmodus eine gesonderte LED-Anzeige 50.

Der praktische Messbetrieb bzw. das Speichern und Übertragen der erfassten Temperaturdaten soll im weiteren anhand der Display-Abbildungen in Fig. 4 bzw. Fig. 5 beschrieben werden, wobei die Fig. 4 die vollständigen Anzeigemöglichkeiten der LCD-Anzeige 46 des vorliegenden Ausführungsbeispiels zeigt. Neben einer dreistelligen Ziffernausgabe existiert nämlich eine Zeile von vier Statusanzeigen 52 für einen aktuellen Betriebs- bzw. Übertragungsmodus, eine Batterie- bzw. Akkuspannungsanzeige 54 sowie eine Temperatureinheits-Anzeige 56 als letzte Stelle, die insbesondere "C" für Grad Celsius bzw. "F" für Grad Fahrenheit ausgeben kann.

Der Messvorgang beginnt durch Abheben des Handmessgerätes 10 von der Adaptereinheit 12, woraufhin das Anzeigedisplay eine über den PC vorbestimmte Programmnummer (z.B. Abfolge von mehreren, vorbestimmten Messschritten) anzeigt. Durch Drücken einer beliebigen der Tasten 42, 44 wird dann bei auf die Hautoberfläche gerichtetem Temperatursensor 34 eine Anzeige für eine laufende Nummer eines aktuellen Messpunktes (Fig. 5: Anzeige für Messpunkt 2) angezeigt, woraufhin dann automatisch in den Messmodus geschaltet wird und eine Temperaturanzeige, in Fig. 4 z.B. 35,8°C, erfolgt.

Wird durch die Bedienperson die erste Taste (Haltetaste) 42 (Hold) betätigt, friert die Anzeige ein. Wird daraufhin innerhalb eines vorbestimmten Zeitfensters von z.B. 2 Sekunden nach Lösen der Haltetaste 42 die zweite Taste (Speichertaste) betätigt, wird der erfasste Temperaturwert in der Speichereinheit 40 gespeichert.

Daraufhin erfolgt die Anzeige des nächsten Messpunktes, z.B. "n03" in Fig. 5.

Zur Anzeige eines aktuellen Temperaturmessmodus blinkt darüber hinaus die LED 46.

Entsprechend dem gewählten Programm werden daraufhin diese Schritte wiederholt, bis der letzte Messpunkt erreicht und gemessen ist. Es erfolgt eine spezielle Quittieranzeige auf dem Display, bis der Kontakt über die Infrarot-Schnittstelle 18 mit der Adaptereinheit 12 hergestellt ist, und nach erfolgter, korrekter Datenübertragung schaltet sich das Handgerät ab.

Über dieses beschriebene, grundsätzliche Bedienverfahren hinaus ermöglicht die vorliegende Ausführungsform weitere Bedienfunktionen, z.B. das Löschen eines Messwertes. Dies kann im vorliegenden Ausführungsbeispiel dadurch gelöst werden, dass beide Tasten 42, 44 gedrückt werden, abgewartet wird, bis eine Statusanzeige 52 aktiviert ist, und daraufhin die Tasten losgelassen werden. Über dieses Verfahren lassen sich dann weitergehende Funktionen steuern, indem nämlich beide Tasten kontinuierlich gehalten werden und suksessive mehrere Statusanzeigen erscheinen; auf diesem Wege lassen sich etwa mehrere Messwerte löschen, das vollständige Messprogramm löschen, oder letzte Schritte rückgängig machen.

Auf Seiten des PC findet dann eine Auswertung der übertragenen Messdaten statt, wobei dies bevorzugt mittels eines unter einem gängigen PC-Betriebssystem lauffähigen Datenbank- bzw. Datenverarbeitungsprogrammes erfolgt. Über die PC-Benutzeroberfläche wird darüber hinaus auch die Möglichkeit zur Programmauswahl, zur Programmdefinition, zur Prozesssteuerung usw. gegeben.

Erfindungsgemäß wird somit sowohl eine einfache, komfortable Erfassung einer Mehrzahl von Temperaturwerten auf einer Oberfläche, z.B. einer Hautoberfläche, erreicht, wobei darüber hinaus eine zuverlässige, benutzerfreundliche Dokumentation und Auswertung der Messung erfolgt.

Während besonders bevorzugt die Übertragung der Messwerte von der Handmesseinheit drahtlos erfolgt, ist es alternativ auch im Rahmen der Erfindung möglich, dass im Handmessgerät die Sequenz von Einzelmesswerten bis zum Programmende gespeichert wird, und dann beim (Wieder-) Verbinden des Handmessgerätes mit der Basisstation, z.B. beim Auflegen des Gerätes, über zugehörige Kontakte bzw. Datenleitungen ein Auslesen der Speichereinheit des Handmessgerätes zur Weiterverarbeitung durch den PC erfolgt.

In der praktischen Erprobung hat sich zudem herausgestellt, dass ein Absolutfehler der Messungen im Bereich ≤ 0,2°C liegt. Damit besteht die Möglichkeit, bei Einhaltung gewisser Rahmenbedingungen die Vorrichtung auch als berührungsloses Fieberthermometer zu benutzen.

Mechanisch sind darüber hinaus sowohl das Handmessgerät als auch die Basiseinheit mittels einer geeigneten Gehäusekonstruktion so realisiert, dass ein einfaches Desinfizieren der Einheiten und mithin eine Eignung für den Einsatz in einer Klinikumgebung sichergestellt sind.

Gemäß einer bevorzugten Weiterbildung der Erfindung ist vorgesehen, dass das Handmessgerät über eine bevorzugt mittels eines Paares von Leuchtdioden (LED) realisierte Fokussiereinheit verfügt, die so ausgebildet ist, dass das sichtbare Licht dieser Leuchtelemente durch eine geeignet vorgesehene Optik in Richtung auf die zu messende Oberfläche gerichtet wird und so fokussiert ist, dass die auf der Oberfläche entstehende Abbildung die Ausdehnung bzw. die Grenzen des Temperaturmessbereiches anzeigen. Auf diese Weise hat die Bedienperson auf einfachem Wege die Möglichkeit, die Temperaturerfassung selektiv auf die konkret interessanten Oberflächenbereiche etwa der Haut oder der Hornhaut eines Auges zu richten, wobei die vorteilhaft vorgesehene Bereichsmarkierung selbst das Messergebnis nicht verfälscht und (da kein Laser) auch bei sensiblen Oberflächen, wie etwa der Hornhaut, unschädlich bleibt.

## Patentansprüche

1. Vorrichtung zur berührungslosen Temperaturerfassung einer Oberflächentemperatur, insbesondere der menschlichen Haut, mit
einer zum berührungslosen Erfassen einer Temperatur als Reaktion auf ein Startsignal sowie zum Ausgeben eines entsprechenden Temperatursignals ausgebildeten, berührungslosen Messeinheit (10),
dadurch gekennzeichnet,
dass die handbetätigt, portabel und netzunabhängig betreibbare Messeinheit (10) zum bevorzugt drahtlosen Übertragen mindestens eines Temperatursignals eine weiter bevorzugt auf Infrarotbasis wirkende Sendereinheit aufweist,
die Sendereinheit (20) zum Zusammenwirken mit einer stationären, mit einer Datenverarbeitungsvorrichtung (14) verbundenen Empfangseinheit (26) ausgebildet ist und die Datenverarbeitungseinheit zum Auswerten einer Mehrzahl von digitalisierten Temperatursignalen als Reaktion auf ein ein Ende eines Messbetriebs bestimmendes Steuersignal ausgebildet ist.

2. Vorrichtung nach Anspruch 1, dadurch gekennzeichnet, dass die berührungslos arbeitende, bevorzugt pyroelektrische oder thermoelektrische Messeinheit eine Speichereinheit (40) zum Speichern einer Mehrzahl von Temperatursignalen aufweist.

3. Vorrichtung nach Anspruch 1 oder 2, dadurch gekennzeichnet, dass die Sendereinheit und die Empfangseinheit zum bidirektionalen Übertragen von Daten ausgebildet sind, wobei die Empfangseinheit zum Rückübertragen eines Betätigungssignals, einer laufenden Nummer für eine aktuelle Messung und/oder eines Messpunktcodes an die berührungslos arbeitende Messeinheit ausgebildet ist.

4. Vorrichtung nach einem der Ansprüche 1 bis 3, dadurch gekennzeichnet, dass die berührungslos arbeitende Messeinheit mit einer mittels einer Ladestation aufladbaren Stromversorgung (39, 41) versehen ist und die Ladestation bevorzugt in die Empfangseinheit (12) integriert ist.

5. Vorrichtung nach einem der Ansprüche 1 bis 4, dadurch gekennzeichnet, dass die berührungslos arbeitende Messeinheit in einem mit einer Hand bedienbaren Gehäuse aufgenommen ist, welches zum Betrieb unter klinischen Bedingungen und zur periodischen Desinfektion geeignet ist.

6. Vorrichtung nach einem der Ansprüche 1 bis 5, gekennzeichnet durch eine zum Zuordnen eines Zeit- und/oder Datumswertes zu einem Temperatursignal oder einer Folge von Temperatursignalen ausgebildeten Zeitgebereinheit.

7. Vorrichtung nach einem der Ansprüche 1 bis 6, dadurch gekennzeichnet, dass die Messeinheit Mittel zum optischen Markieren eines Messbereiches auf der zu messenden Oberfläche aufweist, die eine Strahlungsquelle für sichtbares Licht, insbesondere eine LED, aufweisen, deren Strahlung zum Erzeugen einer in seiner Ausdehnung einer Temperaturmessfläche entsprechenden Markierung auf die Oberfläche projiziert wird.

8. Verfahren zur berührungslosen Temperaturerfassung einer Oberflächentemperatur, insbesondere Verfahren zur Temperaturdatenerfassung auf einer äußeren Oberfläche eines menschlichen Organs, insbesondere zum Betreiben der Vorrichtung nach einem der Ansprüche 1 bis 7, gekennzeichnet durch die Schritte:
- Berührungsloses Erfassen der Oberflächentemperatur als Reaktion auf ein manuelles Startsignal mittels einer pyroelektrischen oder thermoelekrischen Messeinheit,
- drahtloses Übertragen eines der erfassten Temperatur entsprechenden Temperatursignals zu einer Empfangseinheit,
- datentechnisches Auswerten einer Mehrzahl von durch die Empfangseinheit empfangener Temperatursignale als Reaktion auf ein ein Ende eines Messbetriebs anzeigendes Steuersignal.

9. Verfahren nach Anspruch 8, gekennzeichnet durch den Schritt:
- Abspeichern eines Temperatursignals in einer der pyroelektrischen Messeinheit und/oder einer der Empfangseinheit zugeordneten Datenverarbeitungseinheit als Reaktion auf ein manuelles Betätigen eines Bedienknopfes der berührungslos arbeitenden Messeinheit.

10. Verfahren nach Anspruch 8 oder 9, gekennzeichnet durch das fortlaufende oder auf einen vorbestimmten Zeitraum begrenzte Erfassen einer Folge von Temperatursignalen, wobei eine laufende Numerierung einer aktuellen Messung und/oder ein Code für eine Messposition und/oder eine aktuell erfasste Temperatur gespeichert und einer Bedienperson mittels einer Anzeigeeinheit an der Messeinheit optisch signalisiert wird.
